(19) Europäisches Patentamt
European Patent Office
Office européen des brevets

(11) Publication number: 0 061 830 B1

(12) EUROPEAN PATENT SPECIFICATION

(45) Date of publication of patent specification: 29.08.84

(51) Int. Cl.[3]: B 01 J 37/02, B 01 J 37/04, C 07 C 45/35, C 07 C 51/25

(21) Application number: 82300739.8

(22) Date of filing: 15.02.82

# (54) Preparation of composite oxidation catalyst and use thereof for the production of unsaturated aldehydes and acids from propylene and isobutylene.

(30) Priority: 17.02.81 US 235353

(43) Date of publication of application: 06.10.82 Bulletin 82/40

(45) Publication of the grant of the patent: 29.08.84 Bulletin 84/35

(84) Designated Contracting States: BE CH DE FR GB IT LI LU NL SE

(56) References cited:
EP-A-0 000 835
EP-A-0 005 067
US-A-4 040 978
US-A-4 168 246

(73) Proprietor: The Standard Oil Company
Patent & License Division Midland Building
Cleveland Ohio 44115 (US)

(72) Inventor: Callahan, James Louis
R.D. 6 Township Road No. 101
Wooster Ohio 44691 (US)
Inventor: Shaw, Wilfrid Garside
1028 Linden Lane
Lyndhurst Ohio 44124 (US)
Inventor: Grasselli, Robert Karl
150 Greentree Road
Chagrin Falls Ohio 44022 (US)

(74) Representative: Smith, Sydney et al
Elkington and Fife High Holborn House 52/54
High Holborn
London WC1V 6SH (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 061 830 B1

Courier Press, Leamington Spa, England.

## Description

This invention relates to the preparation of a composite oxidation catalyst and to the use thereof.

Several vapour phase processes, such as the production of acrylic acid and the production of maleic anhydride, require two separate catalyst systems, typically in two distinct reactors for optimum results. Commercial processes for the production of acrylic acid require a first reactor and catalyst to convert propylene to acrolein, and a second reactor and catalyst to convert acrolein to acrylic acid. In the same manner, maleic anhydride is prepared by first reacting butene to butadiene, followed by reacting the butadiene to maleic anhydride over a second oxidation catalyst.

It is desirable to have a catalyst that would convert, for example, propylene to acrylic acid in a single stage, thus eliminating a processing step, its energy requirements and its cost. Many different proposals for achieving this goal have been offered, none of which have met with commercial success.

For example, U.S. Patent No. 4,060,545 discloses a fluid bed reactor for the preparation of esters having a single reactor containing two catalysts. Such catalysts can be biased by density etc., so that the first catalyst will have a tendency to be concentrated at the reactor inlet while the second catalyst will be concentrated at the reactor outlet. U.S. Patent No. 3,919,257 describes a process having two catalysts for the preparation of maleic anhydride. A single reactor is utilized having an undivided reaction zone which contains two different catalysts. The ratio of the two catalysts in this mixed bed vary, but in most situations the reaction zone will contain at least about 25% by volume of either catalyst.

British Patent No. 1,256,595 describes layering a single catalyst bed in a fixed bed reactor with both first and second stage oxidation catalysts.

Described as an improvement upon these systems, U.S. Patent No. 3,799,979 discloses a process for converting propylene to acrylic acid in a single reactor having a mixed bed of the two oxidation catalysts that can vary in the range between 8 and 2 and 2 to 8 in parts by volume.

The oxidation catalyst utilized in these reactions are well known in the art. For example, U.S. Patent No. 3,859,358 shows a vapour phase oxidation process utilizing catalysts comprising the oxides of uranium and molybdenum. Similarly, U.S. Patent No. 3,171,859 describes a process for producing unsaturated aldehydes in the presence of a catalyst comprising the oxides of iron, bismuth, phosphorus and molybdenum. Several other catalysts for both first and second stages are disclosed in U.S. Patent No. 3,799,979.

It should be noted from the above that a single catalyst to perform these reactions with a conversion suitable for commercial purposes has not yet been disclosed. While it has been disclosed in U.S. Patent No. 4,060,545 that two catalysts may be composited on a common microsphere, the method of achieving this without having interaction between the two catalysts during the manufacturing thereof has not been known.

It has been discovered that such composite catalysts may be prepared without having detrimental interaction between the components thereof by the use of a non-aqueous organic fluid during preparation. It has further been found that such composite catalysts may be formed with low percentages of one catalyst to the other with excellent dispersion over the particles. It has also been found that high yields of such products as acrylic acid may be achieved in a single reactor utilizing this composite catalyst or mixtures of the composite catalyst with pure first or second stage catalyst.

According to the present invention there is provided a method for preparing a composite oxidation catalyst from a first activated oxidation catalyst and a second catalyst comprising:

a) forming and activating the first oxidation catalyst;

b) forming a second oxidation catalyst;

c) contacting said first catalyst with said second catalyst and a substantially non-aqueous organic fluid to form a composite catalyst; and

d) drying said composite catalyst.

The invention may also be stated as a method for preparing a composite oxidation catalyst comprising:

a) forming and activating the first oxidation catalyst;

b) preparing a solution comprising the salts of the components of said second catalyst and a substantially non-aqueous organic fluid;

c) impregnating the first catalyst with said solution of b);

d) drying said impregnated first catalyst; and

e) calcining said impregnated first catalyst to form and activate the second catalyst within the first catalyst, thus forming a composite catalyst.

The substantially non-aqueous organic fluid utilized in the present invention can be an alcohol such as methanol or ethanol, as well as any non-alcohol organic solvent, examples of which are: glycols containing one to ten carbon atoms, preferably one to four carbon atoms, crown ethers such as 18-crown-6 dimethylformamide, dimethylsulfoxide, ethanol amine, cyclohexane, acetonitrile, acetone and the like. The alcohols such as methanol and ethanol, cyclohexene acetonitrile and acetone are preferred substantially non-aqueous organic fluids.

Substantially non-aqueous means that the organic fluid should contain no more than 10%, preferably less than 5% water.

The method of preparing the composite oxidation catalyst of the present invention can vary in many respects. Common, however, to all these variations are the use of an activated first oxidation catalyst and the substantially non-aqueous organic fluid.

The second catalyst may be incorporated into the particle of the first catalyst using a ground preformed second catalyst or by impregnating the first catalyst with a solution of the salts of the components of the second catalyst followed by drying and activation.

Examples of methods utilizing a preformed second catalyst are given below. The method of forming and activating the first oxidation catalyst, and forming the second oxidation catalyst are both well known in the art, described in the references cited above and do not require to be further described herein.

The first and/or second catalyst may contain a support material.

The second catalyst, after being formed and dried but not yet activated, is ground to a powder. The ground powder is then mixed with the substantially non-aqueous organic fluid to form a suspension. The first activated oxidation catalyst is then mixed with the suspension to impregnate or intrude the second catalyst into the first. After this intrusion or impregnation, the composite catalyst is then dried. The dried composite catalyst can then be activated (calcined) which is usually accomplished by heating the composite catalyst at a temperature of between 200°C to about 600°C, preferably 300°C to 400°C.

The second oxidation catalyst may be activated after forming and prior to grinding at temperatures noted above. After activation, the catalyst is then ground to a powder and mixed with the substantially non-aqueous organic fluid. Grinding may also be accomplished in the presence of the non-aqueous fluid. The suspension solution containing the second oxidation catalyst can then be passed over the first oxidation catalyst to disperse the second catalyst onto the first. This cycle may be repeated several times. Drying may also be accomplished between each cycle. The final composite catalyst may then be activated in the manner described above.

Activated second oxidation catalyst may also be ground and mixed with the first oxidation catalyst in the same manner as the non-activated second catalyst.

Ground activated or non-activated second oxidation catalyst may also be coated upon the particles of the first oxidation catalyst by adding a minimal amount of the non-aqueous organic fluid sufficient to wet the first catalyst and allow coating. Some additional fluid may be added to also wet the ground second catalyst. The composite catalyst is then dried and activated as noted above.

Finally, the first and second catalyst may be combined and ground and then mixed with the non-aqueous organic fluid. A support may be added followed by drying and calcination.

The second catalyst may also be built upon or within the pores of the first catalyst. This is accomplished by preparing a solution of the salts of the components of the second catalyst in the non-aqueous organic fluid. One or more solutions may be prepared containing different components of the second catalyst to avoid any precipitation that may occur when all of the second catalyst components are contained in one solution. After the first catalyst is impregnated with the solution, the composite catalyst is then dried and activated. The first catalyst may also be coated with paraffin or a similar material prior to impregnation to reduce the degree of adverse chemical interaction during the impregnation procedure.

The amount of second catalyst in the composite catalyst according to the invention will typically be less than 10%, preferably in the range of 1—3 wt.%. It has been found, for example, in the preparation of acrylic acid that more than 10% second stage oxidation catalyst results in a decrease in yield of acrylic acid.

The composite catalyst of the present invention may be utilized in fixed, fluid or moving bed reactors.

The following examples further illustrate the invention.

The following experiments show the various methods of preparing a composite catalyst of a known first stage catalyst used to convert propylene to acrolein and a known second stage catalyst used to convert acrolein to acrylic acid.

Since the catalyst utilized and methods of preparing them are well known in the art, a detailed description of such catalysts are not necessary to the present invention. However, the following should be noted:

*First Stage Catalyst* — A supported fixed bed catalyst having 50% active components of potassium, nickel, cobalt, iron, bismuth, phosphorus, molybdenum and oxygen and 50% $SiO_2$ was prepared and activated in the manner set forth in U.S. Patent No. 3,642,930.

*Second Stage Catalyst* — Two second stage oxidation catalysts were utilized, both being prepared according to the procedures set forth in U.S. Patents No. 3,840,595 and No. 4,163,862. Catalyst A and Catalyst B contain molybdenum, vanadium, tungsten, copper, tin, and oxygen, with the amounts of copper and tin being varied between the two catalysts. Both catalysts were prepared from a slurry, and dried to a powder but not activated.

A fixed bed microreactor was charged with 5—6 ml of the appropriate catalyst. A feed mixture of propylene/air/water in the ratio of 1/10/3 was passed over the catalyst charge at the indicated reaction temperature. The feed rate was such that the superficial contact time was approximately 2 seconds in all examples. All examples were proceeded by a pre-run of 30 minutes or more followed by a recovery run of 15 minutes. Percent conversion and per pass conversion to acrylic acid shown in the tables are based upon the propylene fed. The amount of acrylic acid in the reactor effluent was determined by titration of the liquid product and reported as acrylic acid.

Comparative Examples 1—2

Ten-twenty mesh particles of the first stage catalyst were impregnated with an aqueous suspension of finely ground second stage catalyst B which was boiled prior to impregnation. The impregnated particles were first dried on a hot plate with stirring and then oven dried at 150°C for two hours. The particles were finally calcined for two hours at 350°C.

Examples 1—2

Second stage Catalyst B powder was calcined for two hours at 375°C and exposed to the feed mixture for one hour at 325°C. The catalyst was then finally ground and the appropriate amount added to 4 cc of 95% ethanol to form a suspension. The suspension was used to impregnate the first stage catalyst and the mixture was dried on a hot plate with constant stirring. The particles were dried further for two hours at 125°C and then calcined for an additional two hours at 350°C.

The results of the comparative experiments and experiments 1—2 are shown in Table 1. All experiments were run at a temperature of 325°C.

Example 3

Second stage Catalyst A was calcined for two hours at 340°C and then ground with 95% ethanol to produce a fine slurry. The slurry was mixed with first stage catalyst, drained, and dried for two hours at 125°C. The dried particles were calcined for two hours at 340°C and then screened, with the through 10 mesh-retained on 40 mesh (3.5—15 mesh/cm) fraction taken as finished catalyst.

Example 4

Example 4 shows the effect of a reactor having the composite catalyst of Example 3 being blended with pure first stage catalyst. 12.5% of the catalyst of example 3 and 87.5% of the first stage catalyst were mixed and charged to the reactor, thus giving an overall second stage catalyst in the reactor of 1.97 wt.%.

Example 5

Four grams of activated second stage catalyst B was ground extensively in a mortar under 95% ethanol. The slurry was diluted to 100 cc with ethanol and allowed to stand for thirty minutes. The supernatant semi-colloidal dispersion was decanted off and passed over the first stage catalyst supported on a 40 mesh (15 mesh/cm) screen. The colloidal dispersion passing through the screen was retained. The wet particles were dried at 130°C and this cycle was repeated five times. Finally, the treated composite catalyst was dried for two hours at 130°C and calcined an additional two hours at 340°C.

Example 6

A homogeneous composite catalyst containing 2.1 wt.% activated second stage catalyst B, 82.8 wt.% first stage catalyst and 15.1 wt.% $SiO_2$ was prepared by grinding the components and combining in a mortar with enough 95 wt.% ethanol to produce a stiff slurry. The slurry was dried at 135°C for two hours and calcined for two hours at 340°C.

Example 7

A coated composite catalyst was prepared by placing in a wide-mouth jar first stage catalyst particles and a sufficient amount of activated second stage catalyst A fine powder to give a 1 wt.% coating, and rotated to effect a good mixture. Cyclohexane was added drop-wise to the jar with constant rotation until the particles were just wetted-out. Rotation was continued until the particles were well coated. The coated particles were dried for one hour at 200°C.

Example 8

Second stage catalyst B powder was combined with distilled water to form a thick paste. The paste was dried for two hours at 125°C and then calcined in air at 371°C for an additional two hours. The reactor was then charged with a mixture of the catalyst of Example 7 and 2 wt.% of this pure second stage catalyst B.

Example 9

In the same manner of example 7, a composite catalyst having 2.91 wt.% activated second stage catalyst B was coated on the first stage catalyst with cyclohexane. After the composite catalyst was dried for two hours at 130°C, it was calcined for two hours at 340°C.

Example 10

The catalyst of example 9 was diluted with pure first stage catalyst such that the total wt.% second stage catalyst within the reactor was 1.94%.

The results of Examples 3—10, run in the same manner and feeds as Example 2 but varying in reaction temperature are shown in Table II.

Examples 11—14, Multiple Impregnation

Example 11

A solution was made by dissolving phosphomolybdic acid, silico-tungstic acid and $VCl_3$ in warm absolute ethanol. Then $CuAc_2.H_2O$ and $SnCl_2.2H_2O$ were combined and dissolved in absolute ethanol to form a second solution. First stage catalyst were impregnated with the first solution and then the second solution was added to the wet particles. The mixture was first dried with stirring on a hot plate and then oven dried for 2 hours at 130°C. The particles were calcined for 2 hours at 340°C.

Example 12

A first solution was made by dissolving silico-molybdic acid and silico-tungstic acid in acetonitrile. This solution was used to impregnate the first stage catalyst. The impregnated catalyst was dried on a hot plate and then for 15 minutes at 320°C. A second solution was prepared by dissolving $SnCl_2.2H_2O$ and $Cu(Ac)_2.H_2O$ in acetonitrile. The catalyst particles were impregnated with this second solution and dried as before. A third solution was made by dissolving $VCl_3$ in acetonitrile and was used to do a third impregnation of the catalyst particles. The catalyst was finally calcined for 2 hours at 340°C.

Example 13

Silico-molybdic acid and silico-tungstic acid were combined with absolute ethanol to form a first solution. A second solution was prepared by dissolving $SnCl_2.2H_2O$, $Cu(Ac)_2.H_2O$ and $VCl_3$ in acetonitrile, The first stage catalyst particles were impregnated successively with the two solutions with drying at 110°C for 1 hour between impregnations. The catalyst particles were given a final drying and then calcined for 2 hours at 340°C.

Example 14

First stage catalyst particles were added to a wide-mouth jar and cyclohexane was added drop-wise to just fill the catalyst pore volume. Next, all of the dry component salts were added to the jar and the contents were well mixed by rotation. Finally, absolute ethanol was added to the point of just wetting-out the particles. The jar was then rotated for several minutes after which the particles were dried at 130°C for 1 hour and then calcined for 2 hours at 340°C.

The results of Examples 11—14 being run in the same manner as previous examples are contained in Table III.

As can be seen in the Tables, there are many varied methods of preparing the composite catalyst of the present invention with the requirement that a substantially non-aqueous organic fluid be utilized. The intrusion method of preparation provides a dispersion of the second stage catalyst onto the surface of the first stage catalyst particles. While this catalyst does not have optimum physical properties such as attrition resistance, it does show that small quantities of second stage catalysts are sufficient to obtain surprisingly high yields of acrylic acid. Addition of higher quantities of second stage catalysts serves to reduce catalytic efficiency.

As can also be seen in the Tables, the present invention's method of preparing a composite catalyst having low weight percents of the second stage catalyst provides the ability in a single reactor to maximize propylene conversion, minimize waste and secure favorable ratio of acrylic acid to acrolein, within having to layer or grade the separate catalyst particle within the reactor.

Table 1

Preparation of Acrylic Acid Using Composite Catalyst

| Example | Wt. % 2nd. Catalyst | Organic Fluid | % Conversion | Per Pass Conv. Acrylic Acid |
|---|---|---|---|---|
| Comp. 1 | 1.0 | $H_2O$ | 94.0 | 12.8 |
| Comp. 2 | 5.0 | $H_2O$ | 87.6 | 44.4 |
| 1 | 1.0 | Ethanol | 86.5 | 12.9 |
| 2 | 5.0 | Ethanol | 70.2 | 57.2 |

Table 2
Preparation of Acrylic Acid Using Different Types of Composite Catalyst

| Example | 2nd. Catalyst | Wt. % 2nd. Catalyst | Method | Temp. °C | % Conversion | Per Pass Conv. Acrylic Acid |
|---|---|---|---|---|---|---|
| 3 | A | 15.4 | Intrusion | 325 | 69.7 | 42.8 |
| 4 | A | 1.97[(1)] | Intrusion | 375 | 97 | 80.3 |
| 5 | B | 1.0 | Intrusion | 350 | 91 | 50.1 |
| 6 | B | 2.1 | Homogeneous | 400 | 83.8 | 45 |
| 7 | A | 1.0 | Coated | 375 | 97.1 | 68.2 |
| 8 | A | 1.0[(2)] | Coated | 375 | 98.6 | 79.5 |
| 9 | B | 2.91 | Coated | 350 | 89 | 78.1 |
| 10 | B | 1.94[(3)] | Coated | 375 | 97.7 | 83.3 |

(1) 87.5% pure 1st stage + 12.5% composite
(2) 2 wt.% Pure 2nd Stage Added
(3) 66% Pure 1st Stage

Table 3
Preparation of Acrylic Acid Using Impregnated Composite Catalysts

| Example | Organic Fluid | Temp. °C | % Conversion | Per Pass Yield Acrylic Acid |
|---|---|---|---|---|
| 11 | Ethanol | 350 | 51 | 33.6 |
| 12 | Acetonitrile | 375 | 68.9 | 26.7 |
| 13 | Ethanol-Aceto | 350 | 94.9 | 36.1 |
| 14 | Cyclohexane | 375 | 78.5 | 52.2 |

**Claims**

1. A method for preparing a composite oxidation catalyst from a first activated oxidation catalyst and a second catalyst comprising:

a) forming and activating the first oxidation catalyst;

b) forming a second oxidation catalyst;

c) contacting said first catalyst with said second catalyst and a substantially non-aqueous organic fluid to form a composite catalyst; and

d) drying said composite catalyst.

2. The method as claimed in claim 1 characterised in that after forming said second oxidation catalyst in step (b), the catalyst is activated between a temperature of 200—600°C.

3. The method as claimed in claim 1 or claim 2 characterised in that the second catalyst is ground to a powder after being formed in step (b).

4. A method as claimed in claim 3 characterised in that the contacting (c) comprises mixing said ground second catalyst with said non-aqueous fluid to form a solution, and impregnating said solution into said first catalyst.

5. A method as claimed in any of claims 1 to 3 characterised in that the contacting (c) comprises:

e) grinding said second catalyst in the presence of said non-aqueous fluid to form a colloidal dispersion;

f) decanting said colloidal dispersion; and

g) mixing said first catalyst with said colloidal dispersion of second catalyst.

6. A method as claimed in claim 3 charcterised in that the contacting (c) comprises grinding said first catalyst, and mixing ground first catalyst with ground second catalyst and the substantially non-aqueous fluid.

7. A method as claimed in claim 3 characterised in that the contacting (c) comprises coating said first catalyst by mixing said first catalyst with ground second catalyst, and adding a sufficient amount of said non-aqueous fluid to wet and coat the second catalyst onto the first catalyst.

8. A method as claimed in any of claims 1 to 7 characterised in that composite catalyst is after drying activated by heating to a temperature of between 200—600°C.

9. A method as claimed in any of claims 1 to 8 characterised in that the substantially non-aqueous organic fluid is selected from the group consisting of alcohols, acetonitrile, cyclohexene and acetone.

10. A method as claimed in claim 9 characterised in that the substantially non-aqueous organic fluid is ethanol.

11. A method as claimed in claim 9 characterised in that the substantially non-aqueous organic fluid is methanol.

12. A method as claimed in claim 9 characterised in that the substantially non-aqueous fluid is cyclohexane.

13. A method as claimed in any of claims 1 to 12 characterised in that the first oxidation catalyst is supported on a support material.

14. A method as claimed in any of claims 1 to 13 characterised in that the second oxidation catalyst contains a support material.

15. A method for preparing a composite oxidation catalyst from a first activated catalyst and a second catalyst comprising:

a) forming and activating the first oxidation catalyst;

b) preparing a solution comprising the salts of the components of said second catalyst and a substantially non-aqueous organic fluid;

c) impregnating said first oxidation catalyst with said solution of step b) to obtain a composite catalyst;

d) drying said composite catalyst; and

e) activating said composite catalyst.

16. A method as claimed in claim 15 characterised in that two or more solutions of step b) are prepared and wherein drying of step d) occurs after impregnation with each solution.

17. A method as claimed in claim 15 or claim 16 characterised in that the substantially non-aqueous organic fluid is selected for the group consisting of alcohols, acetonitrile, cyclohexene and acetone.

18. A vapour phase process for the preparation of unsaturated aldehydes and acids in a single reactor from propylene or isobutylene comprising passing said propylene or isobutylene, and molecular oxygen over a composite oxidation catalyst, characterised in that the oxidation catalyst has been made by a method as claimed in any of claims 1 to 17.

19. A process as claimed in claim 18 characterised in that the amount of said second catalyst within the reactor is less than 10% by weight, preferably between 1—3% by weight.

**Revendications**

1. Procédé de préparation d'un catalyseur d'oxydation composite à partir d'un premier catalyseur d'oxydation activé et d'un second catalyseur comprenant:

a) la formation et l'activation du premier catalyseur d'oxydation;

b) la formation d'un second catalyseur d'oxydation;

c) la mise en contact dudit premier catalyseur avec ledit second catalyseur et un fluide organique sensiblement non-aqueux pour former un catalyseur composite; et

d) le séchage dudit catalyseur composite.

2. Procédé selon la revendication 1, caractérisé en ce qu'après avoir formé ledit second catalyseur d'oxydation à l'étape (b), on active le catalyseur entre une température de 200°C et de 600°C.

3. Procédé selon l'une des revendications 1 ou 2, caractérisé en ce que le second catalyseur est broyé en une poudre après avoir été formé à l'étape (b).

4. Procédé selon la revendication 3, caractérisé en ce que la mise en contact (c) comprend le mélange dudit second catalyseur broyé avec ledit fluide non-aqueux pour former une solution, et l'imprégnation de ladite solution dans ledit premier catalyseur.

5. Procédé selon l'une des revendications 1 à 3, caractérisé en ce que la mise en contact (c) comprend:

e) le broyage dudit second catalyseur en présence dudit fluide non-aqueux pour former une dispersion colloïdale;

f) la décantation de ladite dispersion colloïdale; et

g) le mélange dudit premier catalyseur à ladite dispersion colloïdale du second catalyseur.

6. Procédé selon le revendication 3, caractérisé en ce que la mise en contact (c) consiste à broyer ledit premier catalyseur et à mélanger le premier catalyseur broyé au second catalyseur broyé et au fluide sensiblement non-aqueux.

7. Procédé selon la revendication 3, caractérisé en ce que la mise en contact (c) consiste à enrober ledit premier catalyseur en mélangeant ledit premier catalyseur au second catalyseur broyé, et à ajouter une quantité suffisante dudit fluide non-aqueux pour mouiller et enrober le second catalyseur sur le premier catalyseur.

8. Procédé selon l'une des revendications 1 à 7, caractérisé en ce que le catalyseur composé est activé, après séchage, en le chauffant à une température comprise entre 200 et 600°C.

9. Procédé selon l'une des revendications 1 à 8, caractérisé en ce que le fluide organique sensiblement non-aqueux est choisi dans le groupe formé par les alcools, l'acétonitrile, le cyclohexène et l'acétone.

10. Procédé selon la revendication 9, caractérisé en ce que le fluide organique sensiblement non-aqueux est de l'éthanol.

11. Procédé selon la revendication 9, caractérisé en ce que le fluide organique sensiblement non-aqueux est du méthanol.

12. Procédé selon la revendication 9, caractérisé en ce que le fluide sensiblement non-aqueux est du cyclohexane.

13. Procédé selon l'une des revendications 1 à 12, caractérisé en ce que le premier catalyseur d'oxydation est supporté sur un matériau-support.

14. Procédé selon l'une des revendications 1 à 13, caractérisé en ce que le second catalyseur d'oxydation contient un matériau-support.

15. Procédé de préparation d'un catalyseur composite d'oxydation à partir d'un premier catalyseur activé et d'un second catalyseur comprenant:

a) la formation et l'activation du premier catalyseur d'oxydation;

b) préparation d'une solution comprenant les sels des composants dudit second catalyseur et un fluide organique sensiblement non-aqueux;

c) l'imprégnation dudit premier catalyseur d'oxydation avec ladite solution de l'étape (b) pour obtenir un catalyseur composite;

d) le séchage dudit catalyseur composite; et

e) l'activation dudit catalyseur composite.

16. Procédé selon la revendication 15, caractérisé en ce que l'on prépare deux solutions ou plus de l'étape (b) et en ce que le séchage de l'étape (d) a lieu après imprégnation avec chaque solution.

17. Procédé selon l'une des revendications 15 ou 16, caractérisé en ce que le fluide organique sensiblement non-aqueux est choisi dans le groupe formé par les alcools, l'acétonitrile, le cyclohexène et l'acétone.

18. Procédé en phase de vapeur pour la préparation d'aldéhydes et acides insaturés dans un seul réacteur à partir de propylène ou d'isobutylène consistant à faire passer ledit propylène ou d'isobutylène et de l'oxygène moléculaire sur un catalyseur d'oxydation composite, caractérisé en ce que le catalyseur d'oxydation a été produit par un procédé selon l'une des revendications 1 à 17.

19. Procédé selon la revendication 18, caractérisé en ce que la quantité dudit second catalyseur dans le réacteur est inférieure à 10% en poids, de préférence comprise entre 1 et 3% en poids.

**Patentansprüche**

1. Verfahren zur Herstellung eines zusammengesetzten Oxidationskatalysators aus einem ersten aktivierten Oxidationskatalysator und einem zweiten Katalysator, das umfaßt:

a) die Bildung und Aktivierung des ersten Oxidationskatalysators;

b) die Bildung eines zweiten Oxidationskatalysators;

c) das Inkontakbringen des ersten Katalysators mit dem zweiten Katalysator und einer im wesentlichen nichtwäßrigen organischen Flüssigkeit unter Bildung eines zusammengesetzten Katalysators; und

d) das Trocknen des zusammengesetzten Katalysators.

2. Verfahren nach Anspruch 1, dadurch gekennzeichnet, daß nach der Bildung des zweiten Oxidationskatalysators in der Stufe (b) der Katalysator bei einer Temperatur zwischen 200 und 600°C aktiviert wird.

3. Verfahren nach Anspruch 1 oder 2, dadurch gekennzeichnet, daß der zweite Katalysator nach der Bildung in der Stufe (b) zu einem Pulver gemahlen wird.

4. Verfahren nach Anspruch 3, dadurch gekannzeichnet, daß das Inkontaktbringen (c) umfaßt das Mischen des gemahlenen zweiten Katalysators mit der nicht-wäßrigen Flüssigkeit unter Bildung einer Lösung und das Imprägnieren des ersten Katalysators mit der Lösung.

5. Verfahren nach einem der Ansprüche 1 bis 3, dadurch gekennzeichnet, daß das Inkontaktbringen (c) umfaßt:

e) das Mahlen des zweiten Katalysators in Gegenwart der nicht-wäßrigen Flüssigkeit unter Bildung einer kolloidalen Dispersion;

f) das Dekantieren der Kolloidalen Dispersion; und

g) das Mischen des ersten Katalysators mit der kolloidalen Dispersion des zweiten Katalysators.

6. Verfahren nach Anspruch 3, dadurch gekennzeichnet, daß das Inkontaktbringen (c) umfaßt das Mahlen des ersten Katalysators und das Mischen des gemahlenen ersten Katalysators mit dem gemahlenen zweiten Katalysator und der im wesentlichen nicht-wäßrigen Flüssigkeit.

7. Verfahren nach Anspruch 3, dadurch gekennzeichnet, daß das Inkontaktbringen (c) umfaßt das Beschichten des ersten Katalysators durch Mischen des ersten Katalysators mit dem gemahlenen zweiten Katalysator und das Zugeben einer ausreichenden Menge der nicht-wäßrigen Flüssigkeit zum Benetzen und Aufbringen des zweiten Katalysators auf den ersten Katalysators in Form einer Schicht.

8. Verfahren nach einem der Ansprüche 1 bis 7, dadurch gekennzeichnet, daß der zusammengesetzt Katalysator nach dem Trocknen durch Erhitzen auf eine Temperatur zwischen 200 und 600°C aktiviert wird.

9. Verfahren nach einem der Ansprüche 1 bis 8, dadurch gekennzeichnet, daß die im wesentlichen nicht-wäßrige organische Flüssigkeit ausgewählt wird aus der Gruppe, die besteht aus Alkoholen, Acetonitril, Cyclohexen und Aceton.

10. Verfahren nach Anspruch 9, dadurch gekennzeichnet, daß die im wesentlichen nicht-wäßrige organische Flüssigkeit Ethanol ist.

11. Verfahren nach Anspruch 9, dadurch gekennzeichnet, daß die im wesentlichen nicht-wäßrige organische Flüssigkeit Methanol ist.

12. Verfahren nach Anspruch 9, dadurch gekennzeichnet, daß die im wesentlichen nicht-wäßrige Flüssigkeit Cyclohexan ist.

13. Verfahren nach einem der Ansprüche 1 bis 12, dadurch gekennzeichnet, daß der erste Oxidationskatalysator auf ein Trägermaterial aufgebracht ist.

14. Verfahren nach einem der Ansprüche 1 bis 13, dadurch gekennzeichnet, daß der zweite Oxidationskatalysator ein Trägermaterial enthält.

15. Verfahren zur Herstellung eines zusammengesetzten Oxidationskatalysators aus einem ersten aktivierten Katalysator und einem zweiten Katalysator, das umfaßt:

a) die Bildung und Aktivierung des ersten Oxidationskatalysators;

b) die Herstellung einer Lösung, welche die Salze der Komponenten des zweiten Katalysators und eine im wesentlichen nicht-wäßrige organische Flüssigkeit enthält;

c) das Imprägnieren des ersten Oxidationskatalysators mit der Lösung der Stufe (b) unter Bildung eines zusammengesetzten Katalysators;

d) das Trocknen des zusammengesetzten Katalysators; und

e) das Aktivieren des zusammengesetzten Katalysators.

16. Verfahren nach Anspruch 15, dadurch gekennzeichnet, daß zwei oder mehr Lösungen der Stuge (b) hergestellt werden und daß die Trocknung der Stufe (d) nach der Imprägnierung mit jeder Lösung erfolgt.

17. Verfahren nach Anspruch 15 oder 16, dadurch gekennzeichnet, daß die im wesentlichen nicht-wäßrige organische Flüssigkeit ausgewählt wird aus der Gruppe, die besteht aus Alkoholen, Acetonitril, Cyclohexen und Aceton.

18. Verfahren zur Herstellung von ungesättigten Aldehyden und Säuren in der Dampfbzw. Gasphase in einem einzigen Reaktor aus Propylen oder Isobutylen, das umfaßt das Drüberleiten von Propylen oder Isobutylen und molekularem Sauerstoff über einen zusammengesetzten Oxidationskatalysator, dadurch gekennzeichnet, daß der Oxidationskatalysator nach einem Verfahren nach einem der Ansprüche 1 bis 17 hergestellt worden ist.

19. Verfahren nach Anspruch 18, dadurch gekennzeichnet, daß die Menge des zweiten Katalysators innerhalb des Reaktors weniger als 10 Gew.-% beträgt, vorzugsweise zwischen 1 und 3 Gew.-% liegt.